# EUROPEAN PATENT APPLICATION

(11) **EP 2 634 261 A1**
(43) Date of publication of application: **04.09.2013**
(21) Application number: 11836466.0
(22) Date of filing: 28.10.2011
(51) Int. Cl.: C12Q 1/60, C12Q 1/32, C12Q 1/44

(54) **METHOD AND KIT FOR MEASURING CHOLESTEROL IN LOW DENSITY LIPOPROTEINS**

(30) Priority: 29.10.2010 JP 2010243569
(71) Applicant: Arkray, Inc., Minami-ku Kyoto-shi Kyoto 601-8045 (JP)
(72) Inventor: IMAI Toshihiro, Kamigyo-ku, Kyoto-shi, Kyoto 602-0008 (JP); HAMA Takashi, Kamigyo-ku, Kyoto-shi, Kyoto 602-0008 (JP)
(74) Representative: Golding, Louise Ann
(86) International application number: PCT/JP2011/074965
(87) International publication number: WO 2012/057333

(57) **Abstract**

A new measurement method by which LDL cholesterol can be measured readily and precisely is provided. The measurement method is for measuring LDL cholesterol in a sample. The method includes the step of subjecting the cholesterol in LDL to an enzyme reaction in the presence of surfactants A1, B1, and B2 in a reaction solution containing the sample treated to eliminate HDL. The surfactant A1 is polyoxyalkylene polycyclic phenyl ether having HLB of 12.5 or less, the surfactant B1 is selected from the group consisting of polyoxyethylene polycyclic phenyl ether having HLB of 12.6 or more, polyoxyethylene distyrenated phenyl ether having HLB of 12.7 or more and 14.5 or less, and polyoxyethylene styrenated phenyl ether having HLB of 12.0 or more and 14.5 or less, and the surfactant B2 is selected from the group consisting of polyoxyethylene lauryl ether having HLB of 16.9 or more, polyoxyethylene myristyl ether, and polyoxyethylene (35-40) octyl phenyl ether.

## Description

### Technical Field

The present invention relates to a method and a kit for measuring cholesterol in low-density lipoproteins (hereinafter referred to as "LDL").

### Background Art

LDL is a lipoprotein having the function of transporting cholesterol synthesized in the liver to peripheral tissues. Cholesterol in LDL (hereinafter also referred to as "LDL cholesterol") is known as one of the causes of arteriosclerosis. Thus, from April 2008, the amount of LDL cholesterol has been in the items to be checked in a medical examination under the Industrial Safety and Health Act.

Examples of LDL cholesterol measurement methods include a method of separating LDL from other lipoproteins (e.g., high-density lipoproteins (hereinafter also referred to as "HDL") and very-low-density lipoproteins (hereinafter also referred to as "VLDL")) by ultracentrifugation and measuring LDL cholesterol only with the use of enzymes, and a method of coloring lipids after separating LDL from other lipoproteins by electrophoresis and measuring the intensity of the color development. However, these methods involve complicated operations, making it difficult to deal with a number of samples by these methods. Therefore, it is difficult to use these methods in clinical tests and the like.

For these reasons, a variety of LDL cholesterol measurement methods using enzymes have been developed. Examples of the measurement methods using enzymes include direct methods by which LDL cholesterol per se is measured directly and elimination methods by which the amount of LDL cholesterol is measured by eliminating cholesterol derived from lipoproteins other than LDL through a first reaction and bringing the remainder of the cholesterol, i.e., LDL cholesterol, into a second reaction.

More specifically, examples of the direct methods include a method in which LDL cholesterol is measured by improving the specificity of enzymes with respect to LDL cholesterol using cyclodextrin and surfactants (Patent Document 1). Further, examples of the elimination methods include a method including preferentially subjecting cholesterol derived from lipoproteins other than LDL to an enzyme reaction using a polyanion and bivalent metal ion, and then measuring LDL cholesterol (Patent Document 2) and a method including subjecting cholesterol derived from lipoproteins other than LDL to an enzyme reaction with calixarene sulfate, polyaniline, and the like bonded to LDL cholesterol to form a soluble complex, and then measuring the LDL cholesterol (Patent Document 3). There is also a method including subjecting cholesterol derived from lipoproteins other than LDL to an enzyme reaction in a buffer solution containing amine and then measuring LDL cholesterol (Patent Document 4).

### Prior Art Documents

### Patent Documents

Patent Document 1: Japanese Patent No. 3091230
Patent Document 2: Japanese Patent No. 3193634
Patent Document 3: Japanese Patent No. 3822340
Patent Document 4: Japanese Patent No. 3058602

### Disclosure of Invention

### Problem to be Solved by the Invention

The present invention provides a new method by which LDL cholesterol can be measured and a kit for use in the measurement.

### Means for Solving Problem

The present invention relates to a method for measuring cholesterol in low-density lipoproteins in a sample. The method includes a step (I) of subjecting the cholesterol in low-density lipoproteins to an enzyme reaction in the presence of surfactants A1, B1, and B2 in a reaction solution containing the sample treated to eliminate high density lipoproteins. The surfactant A1 is a polyoxyalkylene polycyclic phenyl ether having a HLB of 12.5 or less, the surfactant B1 is at least one selected from the group consisting of a polyoxyethylene polycyclic phenyl ether having a HLB of 12.6 or more, a polyoxyethylene distyrenated phenyl ether having a HLB of 12.7 or more and 14.5 or less, and a polyoxyethylene styrenated phenyl ether having a HLB of 12.0 or more and 14.5 or less, and the surfactant B2 is at least one selected from the group consisting of a polyoxyethylene lauryl ether having a HLB of 16.9 or more, a polyoxyethylene myristyl ether, and a polyoxyethylene (35-40) octyl phenyl ether.

Viewed from another aspect, the present invention relates to a kit for use in measurement of cholesterol in low-density lipoproteins. The kit includes: a surfactant A1, the surfactant A1 being a polyoxyalkylene polycyclic phenyl ether having a HLB of 12.5 or less; a surfactant B1, the surfactant B1 being at least one selected from the group consisting of a polyoxyethylene polycyclic phenyl ether having a HLB of 12.6 or more, a polyoxyethylene distyrenated phenyl ether having a HLB of 12.7 or more and 14.5 or less, and a polyoxyethylene styrenated phenyl ether having a HLB of 12.0 or more and 14.5 or less; and a surfactant B2, the surfactant B2 being at least one selected from the group consisting of a polyoxyethylene lauryl ether having a HLB of 16.9 or more, a polyoxyethylene myristyl ether, and a polyoxyethylene (35-40) octyl phenyl ether.

### Effects of the Invention

According to the present invention, a new method for measuring LDL cholesterol can be provided.

### Brief Description of Drawings

FIG. 1 is a graph showing one example of results of Reference Example 1.
FIGS. 2A and 2B each is a graph showing one example of results of Reference Example 3.
FIGS. 3A and 3B each is a graph showing one example of results of Reference Example 4.
FIGS. 4A and 4B each is a graph showing one example of results of Example 1.
FIGS. 5A to 5C each is a graph showing one example of results of Example 2.
FIG. 6A and 6B each is a graph showing one example of the correlation between the measurement values in Example 3 and the measurement values obtained by a conventional method.

### Description of the Invention

The present invention is based upon the findings that LDL cholesterol can be selectively subjected to an enzyme reaction by enzymatically treating, in the presence of the surfactants A1, B1, and B2, a sample containing at least LDL and VLDL, preferably, a sample containing LDL, VLDL, and chylomicron (hereinafter also referred to as "CM"), and more preferably a biological sample treated to eliminate HDL, thereby allowing measurement of LDL cholesterol.

As for why LDL cholesterol can be measured by the LDL cholesterol measurement method of the present invention, the detailed mechanism is not clear. However, it is assumed as follows. That is, the surfactant B1 solubilizes LDL selectively in the presence of the surfactant A1 and the surfactant B2 inhibits solubilization of lipoproteins other than LDL, such as VLDL and CM, in the presence of the surfactants A1 and B1, so that LDL cholesterol can be subjected to an enzyme reaction selectively. It should be noted that the present invention is not limited to this mechanism.

That is, the present invention relates to the following.
[1] A method for measuring cholesterol in low-density lipoproteins in a sample, the method comprising a step (I) of subjecting the cholesterol in the LDL to an enzyme reaction in the presence of surfactants A1, B1, and B2 in a reaction solution containing the sample treated to eliminate HDL,
   wherein the surfactant A1 is a polyoxyalkylene polycyclic phenyl ether having a HLB of 12.5 or less,
   the surfactant B1 is at least one selected from the group consisting of a polyoxyethylene polycyclic phenyl ether having a HLB of 12.6 or more, a polyoxyethylene distyrenated phenyl ether having a HLB of 12.7 or more and 14.5 or less, and a polyoxyethylene styrenated phenyl ether having a HLB of 12.0 or more and 14.5 or less, and
   the surfactant B2 is at least one selected from the group consisting of a polyoxyethylene lauryl ether having a HLB of 16.9 or more, a polyoxyethylene myristyl ether, and a polyoxyethylene (35-40) octyl phenyl ether.
[2] The method for measuring cholesterol in low-density lipoproteins according to [1], wherein the elimination of the HDL includes mixing the surfactant A1, a surfactantA2 and the sample with each other to subject cholesterol in the HDL in the sample to an enzyme reaction, and
   the surfactant A2 is at least one selected from the group consisting of a polyoxyethylene distyrenated phenyl ether having a HLB of 18.0 or more, a polyoxyethylene lauryl ether having a HLB of 16.9 or more, and a polyoxyethylene myristyl ether.
[3] The method for measuring cholesterol in low-density lipoproteins according to [1] or [2], wherein the step (I) includes adding a surfactant B3 to the reaction solution containing the sample treated to eliminate the HDL to subject the cholesterol in the LDL to the enzyme reaction in the presence of the surfactants A1, B1, B2, and B3 in the reaction solution, and
   the surfactant B3 is at least one of a polyoxyethylene alkyl ether sulfate and an alkyl benzene sulfonate.
[4] The method for measuring cholesterol in low-density lipoproteins according to any one of [1] to [3], wherein the polyoxyalkylene polycyclic phenyl ether is a polyoxyethylene polyoxypropylene polycyclic phenyl ether.
[5] The method for measuring cholesterol in low-density lipoproteins according to any one of [1] to [4], wherein the enzyme reaction in the step (I) includes subjecting the low-density lipoproteins in the sample to an enzyme reaction with cholesterol dehydrogenase and cholesterol esterase.
[6] A kit for use in measurement of cholesterol in LDL, the kit comprising:
   a surfactant A1, the surfactant A1 being a polyoxyalkylene polycyclic phenyl ether having a HLB of 12.5 or less;
   a surfactant B1, the surfactant B1 being at least one selected from the group consisting of a polyoxyethylene polycyclic phenyl ether having a HLB of 12.6 or more, a polyoxyethylene distyrenated phenyl ether having a HLB of 12.7 or more and 14.5 or less, and a polyoxyethylene styrenated phenyl ether having a HLB of 12.0 or more and 14.5 or less; and
   a surfactant B2, the surfactant B2 being at least one selected from the group consisting of a polyoxyethylene lauryl ether having a HLB of 16.9 or more, a polyoxyethylene myristyl ether, and a polyoxyethylene (35-40) octyl phenyl ether.
[7] The kit for measuring cholesterol in low-density lipoproteins according to [6], further comprising a surfactant A2, the surfactant A2 being at least one selected from the group consisting of a polyoxyethylene distyrenated phenyl ether having a HLB of 18.0 or more and a polyoxyethylene myristyl ether.
[8] The kit for measuring cholesterol in low-density lipoproteins according to [6] or [7], further comprising a surfactant B3, the surfactant B3 being at least one of a polyoxyethylene alkyl ether sulfate and an alkyl benzene sulfonate.
[9] The kit for measuring cholesterol in low-density lipoproteins according to any one of [6] to [8], further comprising a cholesterol measuring reagent.
[10] The kit for measuring cholesterol in low-density lipoproteins according to [9], wherein the cholesterol measuring reagent includes cholesterol dehydrogenase and cholesterol esterase.
[11] The kit for measuring cholesterol in low-density lipoproteins according to [10], wherein the kit includes a first reagent containing the cholesterol dehydrogenase, a second reagent containing the cholesterol esterase, the surfactants A1 and A2, and a third reagent containing the surfactant B1.

The term "low-density lipoproteins (LDL)" as used herein include both broadly-defined LDL having a specific gravity of 1.006 to 1.063 and narrowly-defined LDL having a specific gravity of 1.019 to 1.063. The term "high-density lipoproteins (HDL)" as used herein refers to lipoproteins having a specific gravity of 1.063 to 1.21.

Samples as used herein may be biological samples, for example. Specific examples of biological samples include bodily fluids and body components of humans and non-human mammals. Examples of bodily fluids and body constituents include, but are not limited to, whole blood, blood plasma, blood serum, spinal fluid, saliva, urine, perspiration, amnionic fluid, pancreatic fluid, lachrymal fluid, and mucosa. In particular, whole blood, blood plasma, and blood serum are preferred. Further, samples to be used may be as is, diluted or separated by centrifugation or the like.

The term "sample treated to eliminate high-density lipoproteins (HDL)" as used herein refers to a sample in which HDL and/or cholesterol derived from HDL (hereinafter may also be referred to as "HDL cholesterol") is eliminated. Examples of such samples may include, but are not particularly limited to, one from which HDL and/or HDL cholesterol is removed or one in which HDL and/or HDL cholesterol is decomposed. Examples of the removal include, but are not particularly limited to, removal by enzyme reaction such as isolating cholesterol from HDL by enzyme reaction and oxidizing/reducing the isolated cholesterol to generate cholestenone. The sample treated to eliminate HDL is preferably one obtained by mixing the surfactants A1 and A2 and a sample with each other to dissolve HDL in the sample, and subjecting HDL cholesterol to an enzyme reaction. Therefore, the sample treated to eliminate HDL contains preferably the surfactants A1 and A2, and more preferably the surfactants A1 and A2, and LDL and/or LDL cholesterol. Further, it is preferable that the sample treated to eliminate HDL is substantially free of HDL and/or HDL cholesterol. The term "be substantially free of HDL and/or HDL cholesterol" means that the amount of HDL and/or HDL cholesterol contained in the sample treated to eliminate HDL is in a range that does not affect the measurement of LDL cholesterol by the measurement method of the present invention.

The term "subjecting LDL cholesterol to an enzyme reaction" as used herein includes, for example, bringing cholesterol and cholesterol measuring enzymes into contact with each other, and preferably includes brining cholesterol measuring enzymes and a color former into contact with cholesterol.

The term "HLB" as used herein is an abbreviation for hydrophilic lipophilic balance, which can be calculated by Griffm's method, for example. Further, HLB values listed in surfactant manufactures' catalogs can also be used.

### [Surfactant A1]

The surfactant A1 is a polyoxyalkylene polycyclic phenyl ether having a HLB of 12.5 or less. In terms of solubilizing HDL more selectively among lipoproteins present in human blood, a polyoxyalkylene polycyclic phenyl ether has a HLB of 12.5 or less, preferably, but is not limited to, 12.3 or more and 12.5 or less, and more preferably about 12.5.

Examples of a polycyclic phenyl of polyoxyalkylene polycyclic phenyl ether include, but are not limited to, a phenyl group having two or more substituents with one aromatic ring and a phenyl group having one or more substituents with two or more aromatic rings. Examples of the substituents with one aromatic ring include, but are not limited to, benzyl and 1-(phenyl)ethyl. Examples of the substituents with two or more aromatic rings include, but are not limited to, naphthyl. Examples of polyoxyalkylene of polyoxyalkylene polycyclic phenyl ether include, but are not limited to, polyoxyethylene, polyoxypropylene, and polyoxybutylene. In particular, one obtained by polymerization of different polyoxyalkylenes is preferred, and one obtained by polymerization of polyoxyethylene and polyoxypropylene is more preferred. Therefore, a polyoxyalkylene polycyclic phenyl ether is preferably a polyoxyethylene polyoxypropylene polycyclic phenyl ether. The method used to polymerize polyoxyethylene and polyoxypropylene is not particularly limited, and either block polymerization or random polymerization may be used, for example.

Specific examples of a polyoxyethylene polyoxypropylene polycyclic phenyl ether having a HLB of 12.5 or less include, but are not limited to, Newcol 2608 F (trade name, HLB: 12.5, manufactured by Nippon Nyukazai Co., Ltd.) and Newcol 707 F (trade name, HLB: 12.5, manufactured by Nippon Nyukazai Co., Ltd.).

### [Surfactant B1]

The surfactant B1 is selected from the group consisting of a polyoxyethylene polycyclic phenyl ether having a HLB of 12.6 or more, a polyoxyethylene distyrenated phenyl ether having a HLB of 12.7 or more and 14.5 or less, and a polyoxyethylene styrenated phenyl ether having a HLB of 12.0 or more and 14.5 or less. They may be used alone or in combination of two or more.

As the surfactant B1, the polyoxyethylene polycyclic phenyl ether has a HLB of 12.6 or more. In terms of being highly capable of solubilizing LDL as compared with VLDL and CM in the presence of the surfactant A1 and solubilizing LDL more selectively, the polyoxyethylene polycyclic phenyl ether has a HLB of preferably 12.6 to 13.6, more preferably 12.6 to 13.3, still more preferably 12.6 to 13.2, and even more preferably about 12.6.

Examples of the polycyclic phenyl of polyoxyethylene polycyclic phenyl ether include, but are not limited to, a phenyl group having two or more substituents with one aromatic ring and a phenyl group having one or more substituents with two or more aromatic rings. Examples of the substituents with one aromatic ring include, but are not limited to, benzyl and 1-(phenyl)ethyl. Examples of the substituents with two or more aromatic rings include, but are not limited to, naphthyl. Examples of a polyoxyalkylene of polyoxyalkylene polycyclic phenyl ether include, but are not limited to, polyoxyethylene, polyoxypropylene and polyoxybutylene. In particular, one obtained by polymerization of different polyoxyalkylenes is preferred, and one obtained by polymerization of polyoxyethylene and polyoxypropylene is more preferred. Therefore, the polyoxyalkylene polycyclic phenyl ether is preferably a polyoxyethylene polyoxypropylene polycyclic phenyl ether. The method used to polymerize polyoxyethylene and polyoxypropylene is not particularly limited, and either block polymerization or random polymerization may be used, for example.

Specific examples of a polyoxyethylene polycyclic phenyl ether having a HLB of 12.6 or more include, but are not limited to, Newcol 2609 (trade name, HLB: 12.6, manufactured by Nippon Nyukazai Co., Ltd.), Newcol 2614 (trade name, HLB: 14.7, manufactured by Nippon Nyukazai Co., Ltd.) and Newcol 610 (trade name, HLB: 13.8, manufactured by Nippon Nyukazai Co., Ltd.).

As the surfactant B1, the polyoxyethylene distyrenated phenyl ether has a HLB of 12.7 or more and 14.5 or less. In terms of being highly capable of solubilizing LDL as compared with VLDL and CM in the presence of the surfactant A1 and solubilizing LDL more selectively, the polyoxyethylene distyrenated phenyl ether has a HLB of preferably about 12.8.

Specific examples of a polyoxyethylene distyrenated phenyl ether having a HLB of 12.7 or more and 14.5 or less include, but are not limited to, Emulgen A60 (trade name, HLB: 12.8, manufactured by Kao Corporation), and EmulgenA90 (trade name, HLB: 14.5, manufactured by Kao Corporation).

As the surfactant B1, the polyoxyethylene styrenated phenyl ether has a HLB of 12.0 or more and 14.5 or less. In terms of being highly capable of solubilizing LDL as compared with VLDL and CM in the presence of the surfactant A1 and solubilizing LDL more selectively, the polyoxyethylene styrenated phenyl ether has a HLB of preferably about 12.7.

Specific examples of a polyoxyethylene styrenated phenyl ether having a HLB of 12.0 or more and 14.5 or less include, but are not limited to, Blaunon TSP16 (trade name, HLB: 12.7, manufactured by Aoki Oil Industrial Co., Ltd.).

### [Surfactant B2]

The surfactant B2 is selected from the group consisting of a polyoxyethylene lauryl ether having a HLB of 16.9 or more, a polyoxyethylene myristyl ether and a polyoxyethylene (35-40) octyl phenyl ether. They may be used alone or in combination of two or more.

As the surfactant B2, the polyoxyethylene lauryl ether polyoxyethylene has a HLB of 16.9 or more. In terms of inhibiting solubilization of VLDL and CM in the presence of the surfactants A1 and B1 and solubilizing LDL more selectively, the polyoxyethylene lauryl ether polyoxyethylene has a HLB of preferably 16.9 to 18.4, more preferably 16.9 to 18.1, still more preferably 16.9 to 17.9, and even more preferably about 16.9. The polymerization degree of a polyoxyethylene lauryl ether polyoxyethylene having a HLB of 16.9 or more is not particularly limited but is, for example, 23 to 50, and preferably 23 to 30. Specific examples of a polyoxyethylene lauryl ether polyoxyethylene having a HLB of 16.9 or more include, but are not limited to, Emulgen 123P (trade name, HLB: 16.9, manufactured by Kao Corporation), Emulgen 130K (trade name, HLB: 18.1, manufactured by Kao Corporation), and Emulgen 150 (trade name, HLB: 18.4, manufactured by Kao Corporation).

As a polyoxyethylene myristyl ether, a polyethylene myristyl ether having a HLB of 18.9 can be used, for example. Specific examples of a polyoxyethylene myristyl ether include, but are not limited to, Emulgen 4085 (trade name, HLB: 18.9, manufactured by Kao Corporation).

In terms of inhibiting solubilization of VLDL and CM in the presence of the surfactants A1 and B1 and solubilizing LDL more selectively, the average polymerization degree of the polyoxyethylene of a polyoxyethylene octyl phenyl ether is 35 to 40, and preferably about 40. Specific examples of a polyoxyethylene (35-40) octyl phenyl ether include, but are not limited to, Triton^{®} X-405 (trade name, polymerization degree: 40, manufactured by Nacalai Tesque Inc.).

### [Surfactant B3]

The surfactant B3 is at least one of a polyoxyethylene alkyl ether sulfate and an alkyl benzene sulfonate. They may be used alone or in combination of two or more. Examples of the salt include, but are not limited to, an ammonium salt, a lithium salt, a sodium salt, a potassium salt, a magnesium salt, and a calcium salt.

In terms of accelerating the speed at which the surfactant B1 solubilizes LDL in the presence of the surfactants A1 and B1, the polymerization degree of polyoxyethylene of polyoxyethylene alkyl ether sulfate is 1 to 10, for example. Examples of a polyoxyethylene alkyl ether sulfate include, but are not limited to, a polyoxyethylene lauryl ether sodium sulfate. Examples of a polyoxyethylene alkyl ether sulfate include, but are not limited to, Emal 20C (trade name, manufactured by Kao Corporation), Newcol 2308SF (trade name, manufactured by Nippon Nyukazai Co., Ltd.) and Newcol 2320SN (trade name, manufactured by Nippon Nyukazai Co., Ltd.).

Examples of an alkyl benzene sulfonate include, but are not limited to, dodecyl (lauryl) benzene sodium sulfate (such as one manufactured by Nacalai Tesque Inc.).

### [SurfactantA2]

The surfactant A2 is selected from the group consisting of a polyoxyethylene distyrenated phenyl ether having HLB of 18.0 or more, polyoxyethylene lauryl ether having a HLB of 16.9 or more and a polyoxyethylene myristyl ether. They may be used alone or in combination of two or more.

As the surfactant A2, the polyoxyethylene distyrenated phenyl ether has a HLB of 18.0 or more. In terms of inhibiting the surfactant A1 from solubilizing lipoproteins other than HDL contained in the sample, the polyoxyethylene distyrenated phenyl ether has a HLB of preferably 18.0 to 19.0, and more preferably about 18.0. Specific examples of a polyoxyethylene distyrenated phenyl ether having a HLB of 18.0 or more include, but are limited to, EmulgenA500 (trade name, HLB: 18.0, manufactured by Kao Corporation).

As the surfactant A2, a polyoxyethylene lauryl ether polyoxyethylene has a HLB of 16.9 or more. In terms of inhibiting the surfactant A1 from solubilizing lipoproteins other than HDL contained in the sample, a polyoxyethylene lauryl ether polyoxyethylene has a HLB of preferably 16.9 to 18.4, more preferably 16.9 to 18.1, still more preferably 16.9 to 17.9, and even more preferably about 16.9. In terms of inhibiting the surfactant A1 from solubilizing lipoproteins other than HDL contained in the sample, the polymerization degree of the polyoxyethylene lauryl etherpolyoxyethylene having a HLB of 16.9 or more is 23 to 50, and preferably 23 to 30, for example. Specific examples of a polyoxyethylene lauryl ether polyoxyethylene having a HLB of 16.9 or more include, but are not limited to, Emulgen 123P (trade name, HLB: 16.9, manufactured by Kao Corporation), Emulgen 130K (trade name, HLB: 18.1, manufactured by Kao Corporation), and Emulgen 150 (trade name, HLB: 18.4, manufactured by Kao Corporation).

Examples of a usable polyoxyethylene myristyl ether include, but are not limited to, a polyethylene myristyl ether having a HLB of 18.9. Specific examples of the polyoxyethylene myristyl ether include, but are not limited to, Emulgen 4085 (trade name, HLB: 18.9, manufactured by Kao Corporation).

### [Cholesterol Measuring Reagent]

A cholesterol measuring reagent is a reagent for use in measurement of cholesterol and includes cholesterol measuring enzymes. The cholesterol measuring enzymes refer to enzymes for use in measurement of cholesterol and examples of which include, but are not limited to, cholesterol dehydrogenase, cholesterol ester hydrolase and coenzymes. Known cholesterol measuring enzymes can be used. As the cholesterol measuring enzymes, it is preferable to use cholesterol dehydrogenase, cholesterol ester hydrolase and coenzymes in combination. The origins of cholesterol dehydrogenase and cholesterol ester hydrolase are not particularly limited. Thus, cholesterol dehydrogenase and cholesterol ester hydrolase may be derived from, for example, microorganisms, animals, or plants, and those produced by gene manipulation may also be used. Further, they may be chemically modified.

Cholesterol ester hydrolase is not particularly limited as along as it is an enzyme capable of hydrolyzing cholesterol ester. Examples of cholesterol ester hydrolase include, but are not limited to, cholesterol esterase and lipoprotein lipase. In particular, cholesterol esterase is preferred. Commercially available cholesterol esterase may also be used and the origin thereof is not particularly limited. Further, in the present invention, two or more kinds of cholesterol ester hydrolase may be used in combination.

Examples of oxidized coenzymes include, but are not limited to, nicotineamide adenine dinucleotide (NAD), nicotineamide adenine dinucleotide phosphate (NADP), thio-NAD, and thio-NADP.

The cholesterol measuring reagent may also include a reduced color former in addition to the cholesterol measuring enzymes. As the reduced color former, one that forms pigment by the action of diaphorase can be used.

Examples of the reduced color former include tetrazolium compounds. Examples of tetrazolium compounds include, but are not limited to, 3-(4,5-dimethyl-2-thiazolyl)-2,5-diphenyl-2H-tetrazolium bromide (MTT), 2-(4-iodophenyl)-3-(4-nitrophenyl)-5-(2,4-disulfophenyl)-2H-tetrazolium monosodium (WST-1), and 2-(4-iodophenyl)-3-(2,4-dinitrophenyl)-5-(2,4-disulfophenyl)-2H-tetrazolium monosodium (WST-3).

### [LDL Cholesterol Measurement Method]

### (LDL Cholesterol Measurement Method According to First Aspect)

The LDL cholesterol measurement method according to the first aspect of the present invention includes the step (I) of subjecting cholesterol in LDL to an enzyme reaction in the presence of the surfactants A1, B1, and B2 in a reaction solution containing the sample treated to eliminate HDL. Hereinafter, the LDL cholesterol measurement method according to the first aspect will be described.

In the step (I), cholesterol in LDL in a sample is measured by subjecting the cholesterol in LDL to an enzyme reaction in the presence of the surfactants A1, B1, and B2 in a reaction solution containing the sample treated to eliminate HDL.

As the sample, one treated to eliminate HDL is used. Examples of the HDL elimination treatment include mixing the surfactants A1 and A2 and the sample with each other to dissolve HDL in the sample, thereby subjecting cholesterol derived from HDL to an enzyme reaction.

The concentration of the surfactant B1 in the reaction solution is not particularly limited but is 0.44 to 0.97 wt%, for example. In terms of improving the surfactant B1's sole capability of solubilizing LDL as compared with VLDL and CM in the presence of the surfactant A1 and solubilizing LDL more selectively, the concentration of the surfactant B1 in the reaction solution is preferably 0.62 to 0.79 wt%. The concentration of the surfactant B2 in the reaction solution is not particularly limited but is 0.01 to 0.04 wt%, for example. In terms of inhibiting solubilization of VLDL and CM in the presence of the surfactants A1 and B1 and solubilizing LDL more selectively, the concentration of the surfactant B2 in the reaction solution is preferably 0.018 to 0.03 wt%. The weight percent ratio of the surfactant B1 to the surfactant B2 (B1:B2) in the reaction solution is not particularly limited but is 44:1 to 97:4, and preferably 31:1 to 79:3, for example.

The step (I) may include adding the surfactants A1, B1, and B2 to the reaction solution containing the sample treated to eliminate cholesterol derived from HDL by solubilization of HDL. If the sample treated to eliminate HDL contains the same surfactant as the surfactant A1, the surfactant contained in the sample may be used as the surfactant A1 without adding the surfactant A1, or the surfactant A1 may be added additionally in the step (I). If the sample treated to eliminate HDL contains the same surfactant as the surfactant B2, the surfactant contained in the sample may be used as the surfactant B2 without adding the surfactant B2, or the surfactant B2 may be added additionally in the step (I).

In the step (I), a cholesterol measuring reagent may be added to the reaction solution. The cholesterol measuring reagent may include, for example, cholesterol measuring enzymes such as cholesterol dehydrogenase and cholesterol ester hydrolase, a coenzyme, a reduced color former, diaphorase, and the like. If the cholesterol measuring reagent includes a reduced color former and diaphorase, reduced color former-derived pigment produced by a reaction through a reduced coenzyme and diaphorase allows detection with longer wavelength visible light.

The concentration of cholesterol dehydrogenase is not particularly limited but is, for example, 1.74 to 4.35 U/mL. It is preferable that the concentration is 2.9 to 4.35 U/mL because the speed at which the reaction reaches the endpoint becomes the highest when the cholesterol concentration in the reaction solution is 0.012 mM. The concentration of cholesterol ester hydrolase in the reaction solution is not particularly limited but is, for example, 0.19 to 0.58 U/mL. It is preferable that the concentration is 0.29 to 0.58 U/mL because the speed at which the reaction reaches the endpoint becomes the highest when the cholesterol concentration in the reaction solution is 0.012 mM. In connection with the range of the cholesterol dehydrogenase concentration, the concentration of coenzyme in the reaction solution is, for example, 0.29 to 0.97 mM, and preferably 0.73 to 0.97mM.

The concentration of diaphorase in the reaction solution is not particularly limited but is, for example, 1.74 to 4.35 U/mL, and preferably 2.9 to 4.35 U/mL. The concentration of reduced color former is, for example, 0.10 to 0.29 mM, and preferably 0.19 to 0.29 mM.

It is preferable that the reaction in the step (I) takes place in a buffer solution in order to maintain the buffer capability in the reaction solution and to retain the pH as set. Examples of buffering agents to be used in the buffer solution include Good's buffering agents, phosphate buffering agents and Tris buffering agents. Examples of Good's buffering agents include, but are not limited to, N,N-bis(2-hydroxyethyl)-2-aminoethanesulfonic acid (BES), 2-morpholinoethanesulfonic acid (MES), bis(2-hydroxyethyl)iminotris(hydroxymethyl)methane (Bis-Tris), N-(2-acetamide)iminodiacetic acid (ADA), piperazine-N,N'-bis(2-ethanesulfonic acid) (PIPES), N-(2-acetamide)-2-aminoethanesulfonic acid (ACES), 3-morpholino-2-hydroxypropanesulfonic acid (MOPSO), 3-morpholinopropanesulfonic acid (MOPS), N-[tris(hydroxymethyl)methyl]-2-aminoethanesulfonic acid (TES), 2-[4-(2-hydroxyethyl)-1-piperazinyl]ethanesulfonic acid (HEPES), N-[tris(hydroxymethyl)methyl]glycine (Tricine), N,N-bis(2-hydroxyethyl)glycine (Bicine), N-tris(hydroxymethyl)methyl-3-aminopropanesulfonic acid (TAPS), N-cyclohexyl-2-aminoethanesulfonic acid (CHES), N-cyclohexyl-3-amino-2-hydroxypropane sulfonic acid (CAPSO), and N-cyclohexyl-3-aminopropanesulfonic acid (CAPS). The concentration may be adjusted so that the buffer capability in the reaction solution can be maintained and the pH as set is retained. The pH of the reaction solution may be adjusted as appropriate in view of the pH stability and reaction suitability of enzymes to be used. In this measurement method, the pH is, for example, 6.5 to 10.0, and preferably 7.5 to 8.0.

In the step (I), it is preferable that the surfactant B3 is added to the reaction solution to carry out the enzyme reaction in the presence of the surfactants A1, B1, B2, and B3. This can promote, for example, selective solubilization of LDL, and more preferably can further suppress the influence of VLDL. The concentration of the surfactant B3 in the reaction solution is not particularly limited but is, for example, 0.03 to 0.07 wt%. In term of further accelerating solubilization of LDL, the concentration of the surfactant B3 in the reaction solution is preferably 0.04 to 0.06 wt%.

The reaction temperature in the step (I) is not particularly limited but is generally about 37°C when measuring human blood components, particularly biological items. The reaction time is not particularly limited but is, for example, 180 to 300 seconds, and preferably 240 to 270 seconds because LDL cholesterol can be quantified adequately with the present reaction system even if the concentration of LDL cholesterol in human blood is around 500 mg/dL.

LDL cholesterol can be measured by determining the absorbance of the reaction solution, for example. To determine the absorbance, the absorbance of pigment produced by a hydrogen peroxide measuring reagent may be determined.

### (LDL Cholesterol Measurement Method According to Second Aspect)

The LDL cholesterol measurement method according to the second aspect of the present invention includes a step (i) of mixing the sample, the surfactants A1 and A2 and a cholesterol measuring reagent with each other to subject cholesterol in HDL in the sample to an enzyme reaction, and a step (ii) of measuring cholesterol in LDL in the sample by subjecting the cholesterol in LDL to an enzyme reaction in the presence of the surfactants B1 and B2 in the reaction solution after the step (i).

As for why LDL cholesterol can be measured by the LDL cholesterol measurement method according to the present aspect, i.e., by selectively subjecting HDL cholesterol and LDL cholesterol to an enzyme reaction in this order, the detailed mechanism is not clear. However, it is assumed as follows. That is, the surfactant A1 solubilizes HDL selectively as compared with lipoproteins other than HDL, such as LDL, VLDL and CM, and the surfactant A2 inhibits solubilization of lipoproteins other than HDL in the presence of the surfactant A1, so that HDL cholesterol can be subjected selectively to an enzyme reaction. Furthermore, after this reaction, the surfactant B1 solubilizes LDL selectively as compared with lipoproteins other than LDL, such as VLDL and CM, in the reaction solution after the reaction, and the surfactant B2 inhibits solubilization of lipoproteins other than LDL in the presence of the surfactant B1, so that LDL cholesterol can be subjected selectively to an enzyme reaction. When the same surfactant as the surfactant A2 is used as the surfactant B2, there is no need to add the surfactant B2 additionally in the step (ii). It should be noted that the present invention is not limited to this mechanism.

Hereinafter, the LDL cholesterol measurement method according to the second aspect will be described.

### [Step (i)]

First, in the step (i), a sample, the surfactants A1 and A2, and a cholesterol measuring reagent are mixed with each other to subject cholesterol in HDL in the sample to an enzyme reaction.

The order in which the sample, the surfactants A1 and A2 and the cholesterol measuring reagent are mixed is not particularly limited. After mixing the sample with part of the cholesterol measuring reagent in advance, the remainder of the cholesterol measuring reagent and the surfactants A1 and A2 may be added to the mixture. Or, the sample, the surfactants A1 and A2 and the cholesterol measuring reagent may be mixed with each other at the same time.

The concentration of the surfactant A1 in the reaction solution in the step (i) (HDL elimination step) is not particularly limited but is, for example, 0.22 to 0.30 wt%. In terms of solubilizing HDL more selectively, the concentration of the surfactant A in the reaction solution is preferably 0.22 to 0.28 wt%. The concentration of the surfactantA2 in the reaction solution is not particularly limited but is, for example, 0.02 wt% or more. In terms of inhibiting the solubilization of lipoproteins other than HDL while suppressing the influence of the surfactant A1 on the solubilization of HDL, the concentration of the surfactantA2 in the reaction solution is preferably 0.04 to 0.10 wt%, and more preferably 0.06 to 0.09 wt%. The weight ratio of the surfactant A1 to the surfactant A2 (A1:A2) in the reaction solution is not particularly limited but is, for example, 11:1 to 15:1. In terms of inhibiting the solubilization of lipoproteins other than HDL while suppressing the influence of the surfactant A1 on the solubilization of HDL, and solubilizing HDL more selectively, the weight ratio of the surfactant A1 to the surfactant A2 in the reaction solution is preferably 10:2.4 to 10:3.7.

The composition of the cholesterol measuring reagent and the concentration of each component are the same as those in the step according to the first aspect. Further, it is preferable that the reaction in the step (i) takes place in a buffer solution in order to maintain the buffer capability in the reaction solution and to retain the pH as set. The same buffering agents and pH as those explained in the first aspect can be used.

The reaction temperature in the step (i) is not particularly limited but is generally about 37°C when measuring human blood components, particularly biological items. The reaction time is not particularly limited but is, for example, 200 to 270 seconds, and preferably 240 to 270 seconds because HDL cholesterol can be adequately eliminated with the present reaction system even if the concentration of HDL cholesterol in human blood is around 150 mg/dL.

### [Step (ii)]

Next, in the step (ii), cholesterol in LDL in the sample is measured by subjecting the cholesterol in LDL to an enzyme reaction in the presence of the surfactants B1 and B2 in the reaction solution after the step (i).

The step (ii) may include adding the surfactants B1 and B2 to the reaction solution after the step (i). Further, when the same surfactant as the surfactant A2 is used as the surfactant B2 in the step (ii), the surfactant B2 may be additionally added in the step (ii) or the surfactant A2 used in the step (i) may be used as the surfactant B2 without additionally adding the surfactant B2.

In the reaction solution, the concentration of each of the surfactants B1 and B2 and the weight percent ratio of the surfactant B1 to the surfactant B2 are the same as those in the step (I) according to the first aspect. The weight percent ratio of the surfactant A1 to the surfactant B1 to the surfactant B2 (A1:B1:B2) is not particularly limited but is, for example, 19:44:1 to 25:97:4, and preferably 19:62:2 to 23:79:3.

In the step (ii), a cholesterol measuring reagent may be additionally added or the cholesterol measuring reagent used in the step (i) may be used without additionally adding the reagent.

In the step (ii), it is preferable to further add the surfactant B3 to the reaction solution after the step (i) to carry out the enzyme reaction in the presence of the surfactants B1, B2, and B3. This can promote, for example, selective solubilization of LDL, and more preferably this can further suppress the influence of VLDL. The concentration of the surfactant B3 in the reaction solution is the same as that in the step (I) according to the first aspect.

The reaction temperature and the reaction time in the step (ii) are the same as those in the step (I) according to the first aspect.

LDL cholesterol can be measured by determining the absorbance of the reaction solution, for example. To determine the absorbance, the absorbance of pigment produced by a hydrogen peroxide measuring reagent may be determined, for example.

### [Kit for Measuring LDL Cholesterol]

Viewed from one aspect, the kit for measuring LDL cholesterol of the present invention is a kit for use in measurement of cholesterol in LDL. The kit includes the surfactants A1, B1, and B2 (hereinafter also referred to as "the kit of the present invention"). The kit of the present invention can be used in the LDL cholesterol measurement method of the present invention. The surfactants A1, B1, and B2 are the same as those in the LDL cholesterol measurement method of the present invention.

The kit of the present invention may include a cholesterol measuring reagent. The cholesterol measuring reagent is the same as that in the LDL cholesterol measurement method of the present invention.

The kit of the present invention may include the surfactant B3. The surfactant B3 is the same as that in the LDL cholesterol measurement method of the present invention.

The kit of the present invention may include the surfactant A2. The surfactant A2 is the same as that in the LDL cholesterol measurement method of the present invention.

Viewed from another aspect, the kit of the present invention is a kit for use in measurement of cholesterol in LDL, which include the surfactants A1, A2, and B1. The kit for measuring LDL cholesterol according to this aspect may include the surfactant B2 or may include the surfactant B3 and a cholesterol measuring reagent. The surfactants A1, A2, B1, B2, and B3 and the cholesterol measuring reagent are the same as those in the LDL cholesterol measuring method of the present invention.

Examples of the kit of the present invention include a two-reagent system kit, a three-reagent system kit, and a four-reagent system kit.

When the kit is in the form of a two-reagent system kit including cholesterol dehydrogenase, cholesterol dehydrogenase is preferably contained only in the first reagent. Cholesterol esterase may be contained in, for example, at least one of the first and second reagents but is preferably contained only in the second reagent. Both the surfactants A1 and A2 are preferably contained only in the first reagent. The surfactants B1, B2, and B3 are preferably contained only in the second reagent.

When the kit is in the form of a three-reagent system kit, cholesterol dehydrogenase and the surfactants A1 and A2 may be contained in at least one of the first reagent, the second reagent, or the first reagent only as the combination of the reagents whose priority is given to the first reaction, for example. Cholesterol esterase may be contained in at least one of the first reagent, the second reagent, and the third reagent, and is preferably contained only in the reagent whose priority is given to the second reaction. The surfactants B1, B2, and B3 may be contained in at least one of the second reagent, the third reagent, or the third reagent only as the combination of the reagents whose priority is given to the second reaction.

Examples of preferred forms of the measurement kit include a measurement kit having a first reagent containing cholesterol dehydrogenase, a second reagent containing cholesterol esterase, the surfactants A1 and A2, and a third reagent containing the surfactant B1.

The kit of the present invention preferably includes an instruction manual describing the way to measure LDL cholesterol using the above reagents. Even when the instruction manual is not packed with the measurement kit of the present invention but is provided on the web, it is still within the scope of the kit of the present invention.

In the kit of the present invention, a reagent pack may accommodate the cholesterol measuring reagents, the surfactants A1, A2, and B1, etc. The reagent pack is preferably in the form of a disposable cartridge and more preferably includes two or more types of vessels in which the reagents can be placed. In the kit of the present invention, it is preferable that the first reagent, the second reagent, and the third reagent of the reagent pack are individually placed in different vessels. The reagent pack may further include, for example, a reaction vessel, a specimen vessel, a dispensation chip, a reaction optical cell, and a disposal vessel. By using such a reagent pack, the LDL cholesterol measurement method of the present invention can be carried out more readily and be applied to an automated device with extreme ease.

Hereinafter, the present invention will be described in more detail with reference to Reference Examples and Examples. It should be noted that the present invention is not limited to the following Examples.

In the following Examples, the following abbreviations are used.
CHDH: cholesterol dehydrogenase (trade name: CHDH "Amano" 5, manufactured by Amano Enzyme Co., Ltd.)
Diaphorase: dihydrolipoamide dehydrogenase (trade name: Diaphorasel, manufactured by Unitica Ltd.)
BES: N,N-bis(2-hydroxyethyl)-2-aminoethanesulfonic acid (manufactured by Dojindo Laboratories Inc.)
CE: cholesterol esterase (trade name: COE-311, manufactured by Toyobo Co., Ltd.) MTT: 3-(4,5-dimethylthiazole-2-yl)-2,5-diphenyltetrazolium bromide (manufactured by Dojindo Laboratories Inc.)
BSA: bovine serum albumin (manufactured by Sigma Inc.)
NAD: nicotine amide adenine nucleotide (manufactured by Oriental Yeast Co., Ltd.)

### Examples

### (Reference Example 1)

The reagents 1-1 to 1-4 and 2 as presented in Table 1 below were used to measure LDL cholesterol in samples. The samples used were each obtained by adding to human serum free of lipoproteins VLDL and LDL recovered from human serum by ultracentrifugal fraction and diluting the resultant 20 times with the reagents 1-3 (LDL concentration: 83 mg/dL, VLDL concentration: 61 mg/dL).

To 20 µL of each sample, 90 µL of reagent 1-1, 30 µL of reagent 1-2, and 15 µL of reagent 1-4 were added in order, and they were reacted with each other for 240 seconds at 37°C. Then, the reagents 2 containing surfactants presented in Table 2 below were added to the samples, respectively, in amount of 15 µL, and the absorbance was measured using a general purpose automatic analyzer (trade name: BioMajesty-8, manufactured by JEOL Ltd.) and measurement values of LDL cholesterol were obtained. Table 2 below provides the results.

| (Table 1) | | Concentration at the time of addition | Final concentration |
|---|---|---|---|
| Reagent 1-1 | CHDH | 5 U/mL | 4 U/mL |
| | Diaphorase | 5 U/mL | 4 U/mL |
| | BES (pH 7.0) | 378 mM | 300 mM |
| Reagent 1-2 | surfactant A1 *¹ | 0.24 wt% | 0.19 wt% |
| | surfactant A2 *² | 0.07 wt% | 0.06 wt% |
| | CE | 3 U/mL | 2.4 U/mL |
| | BES (pH 7.0) | 378 mM | 300 mM |
| Reagent 1-3 | Tris-HCl (pH 9.0) | 400 mM | 44.7 mM |
| Reagent 1-4 | phosphate buffer (pH 6.0) | 20 mM | 1.76 mM |
| | MTT | 0.22 mM | 0.17 mM |
| | NAD | 1.1 mM | 1.9 mM |
| Reagent 2 | surfactant | | |

| | | | |
|---|---|---|---|
| *¹ polyoxyalkylene polycyclic phenyl ether: Newcol 2608 F (trade name: manufactured by Nippon Nyukazai Co., Ltd., HLB 12.5, the product includes oxyethylene and oxypropylene as oxyalkylene) *² polyoxyethylene distyrenated phenyl ether: Emulgen A500 (trade name: manufactured by Kao Corporation, HLB 18.0) | | | |

**(TABLE 2)**

| Reagent 2 (surfactant) | | | Concentration (wt%) | LDL measurement value (mg/dL) | Divergence From assayed value |
|---|---|---|---|---|---|
| Composition | HLB | Trade name | | | |
| polyoxyethylene polycyclic phenyl ether | 12.6 | Newcol 2609 *¹ | 5 | 80.1 | -2.9 |
| polyoxyethylene distyrenated phenyl ether | 12.8 | Emulgen A60 *² | 5 | 81.5 | -1.5 |
| polyoxyethylene distyrenated phenyl ether | 12.8 | Emulgen A60 *² | 2 | 72.6 | -10.4 |
| polyoxyethylene styrenated phenyl ether | 12.7 | Blaunon TSP-16 *³ | 5 | 81.0 | -2.0 |
| polyoxyethylene styrenated phenyl ether | 12.7 | Blaunon TSP-16 *³ | 2 | 89.0 | 6.0 |
| polyoxyethylene (10) octyl phenyl ether | - | Triton X-100 | 2 | 90.8 | 7.8 |
| polyoxyethylene (10) octyl phenyl ether | - | Triton X-100 | 5 | 112.4 | 29.4 |
| polyethylene glycol monolaurate | 13.7 | Emanon 1112 *² | 2 | 105.8 | 22.8 |
| polyethylene glycol monolaurate | 13.7 | Emanon 1112 *² | 5 | 121.2 | 38.2 |
| polyethylene (23) lauryl ether | 16.9 | Birji 35 *⁴ | 2 | 110.4 | 27.4 |
| polyoxyalkylene alkyl ether | 12.7 | Emulgen MS-110 *² | 2 | 123.4 | 40.4 |
| polyoxyalkylene alkyl ether | 12.7 | Emulgen MS-110 *² | 5 | 126.4 | 43.4 |
| polyoxyethylene alkyl ether | 13.3 | Emulgen 709 *² | 2 | 127.2 | 44.2 |
| Polyoxyethylene alkyl ether | 13.3 | Emulgen 709 *² | 5 | 131.1 | 48.1 |
| polyoxyethylene (12) nonyl phenyl ether | 13.0 | Igepal CO-630 *⁵ | 2 | 125.9 | 42.9 |

| | | | | | |
|---|---|---|---|---|---|
| *¹ manufactured by Nippon Nyukazai Co., Ltd. *² manufactured by Kao Corporation *³ manufactured by Aoki Oil Industrial Co., Ltd *⁴ manufactured by SIGMACo. *⁵ manufactured by Aldrich Co. | | | | | |

As can be seen from Table 2 above, when a polyoxyethylene polycyclic phenyl ether having a HLB of 12.6, a polyoxyethylene distyrenated phenyl ether having a HLB of 12.8, or a polyoxyethylene styrenated phenyl ether having a HLB of 12.7 was used as the surfactant of the reagent 2, the divergence of the obtained measurement value of LDL from the assayed value (83 mg/dL) was small. Further, even when a sample with a high LDL concentration (LDL concentration: 141 mg/dL) was used, the divergence from the assayed value was similarly small (data not shown). Therefore, it was found that LDL could be solubilized selectively in the presence of the surfactant A1 by using a polyoxyethylene polycyclic phenyl ether having a HLB of 12.6, a polyoxyethylene distyrenated phenyl ether having a HLB of 12.8, or a polyoxyethylene styrenated phenyl ether having a HLB of 12.7.

### (Reference Example 2)

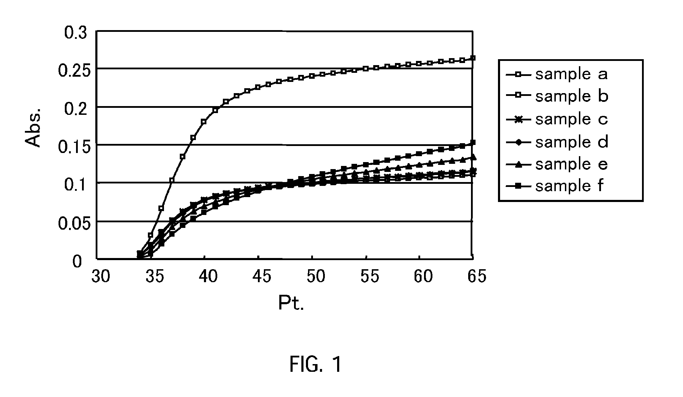

A polyoxyethylene distyrenated phenyl ether having a HLB of 12.8 (trade name: EmulgenA60, manufactured by Kao Corporation, concentration: 8 wt% (final concentration: 0.71 wt%)) as the surfactant of the reagent 2 and samples a to f, whose compositions are as presented in Table 3 below, were used to determine the absorbance in the same manner as in Reference Example 1. The results are shown in FIG. 1. In FIG. 1, the vertical axis indicates absorbance and the horizontal axis indicates measuring time, and 1 Pt. corresponds to 9 seconds.

| (Table 3) | LDL (mg/dL) | VLDL (mg/dL) |
|---|---|---|
| sample a | 51 | - |
| sample b | 136 | - |
| sample c | 52 | 19 |
| sample d | 52 | 60 |
| sample e | 51 | 104 |
| sample f | 50 | 160 |

As can be seen from FIG. 1, in the case of the samples c and d each having a VLDL concentration of less than 100 mg/dL, it was shown that LDL could be solubilized selectively by subjecting LDL to an enzyme reaction in the presence of the surfactants A1 and B1. On the other hand, in the case of the samples e and f each having a VLDL concentration of more than 100 mg/dL, it was shown that not only LDL but also VLDL were solubilized with the passage of the measuring time.

### (Reference Example 3)

Regents 1-1 to 1-4 as presented in Table 4 below were used to eliminate HDL cholesterol in samples. The samples used were each obtained by adding a physiological saline solution to a HDL preparation recovered from human serum by ultracentrifugal fraction and concentrated adequately (HDL concentration: 160 mg/dL).

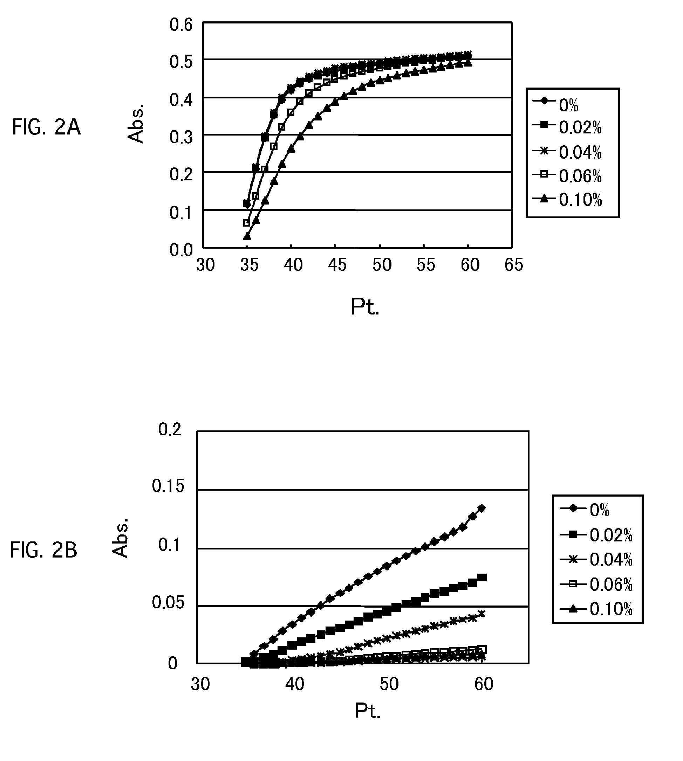

To each sample, the reagents 1-1, 1-2, and 1-4 were added in order and they were reacted with each other for 240 seconds at 37°C. Then, the absorbance was determined using a general purpose automatic analyzer (trade name: BioMajesty-8, manufactured by JEOL Ltd.) and measurement values of HDL cholesterol were obtained. FIG. 2A shows the results.

Further, in order to check whether or not LDL was solubilized at the time of eliminating HDL, the measurement was performed in the same manner as above except for using samples each obtained by adding a physiological saline solution to a LDL preparation recovered from human serum by ultracentrifugal fraction and concentrated adequately (LDL concentration: 347 mg/dL) and for measuring LDL cholesterol. FIG. 2B shows the results.

| (Table 4) | | Concentration at the time of addition | Final concentration |
|---|---|---|---|
| Reagent 1-1 | CHDH | 7.5 U/mL | 4.4 U/mL |
| | Diaphorase | 7.5 U/mL | 4.4 U/mL |
| | BES (pH 7.0) | 300 mM | 174 mM |
| Reagent 1-2 | surfactant A1 *¹ | 1.1 wt% | 0.22 wt% |
| | surfactant A2 *² | 0 to 0.5 wt% | 0 to 0.10 wt% |
| | CE | 3 U/mL | 154 mM |
| | BES (pH 7.0) | 800 mM | 51 mM |
| Reagent 1-3 | Tris-HCl (pH 9.0) | 400 mM | 3.9 mM |
| Reagent 1-4 | phosphate buffer (pH 6.0) | 20 mM | 0.2 mM |
| | MTT | 1.0 mM | 0.48 mM |
| | NAD | 2.5 mM | 0.44 mM |

| | | | |
|---|---|---|---|
| *¹ polyoxyalkylene polycyclic phenyl ether: Newcol 2608 F (trade name: manufactured by Nippon Nyukazai Co., Ltd., HLB 12.5, the product includes oxyethylene and oxypropylene as oxyalkylene) *² polyoxyethylene distyrenated phenyl ether: Emulgen A500 (trade name: manufactured by Kao Corporation, HLB 18.0) | | | |

FIG. 2A is a graph showing one example of results of eliminating HDL using the surfactantA2 in various concentrations (final concentration: 0 wt%, 0.02 wt%, 0.04 wt%, 0.06 wt%, and 0.11 wt%) and FIG. 2B is a graph showing one example of results of checking, by using the surfactant A2 in various concentrations, whether or not LDL was solubilized. In FIGS. 2A and 2B, the vertical axis indicates absorbance and the horizontal axis indicates measuring time. In FIGS. 2A and 2B, 1 Pt. corresponds to 9 seconds. As can be seen from FIG. 2A, by enzymatically treating the sample containing HDL in the presence of the surfactants A1 and A2, HDL contained in the sample was brought into a reaction with enzymes and thus was eliminated almost entirely. Further, as can be seen from FIG. 2B, solubilization of LDL was suppressed adequately in the presence of the surfactants A1 and A2. In particular, solubilization of LDL could be suppressed more effectively when the concentration of the surfactant added was 0.3 wt% (final concentration: 0.06 wt%).

### (Reference Example 4)

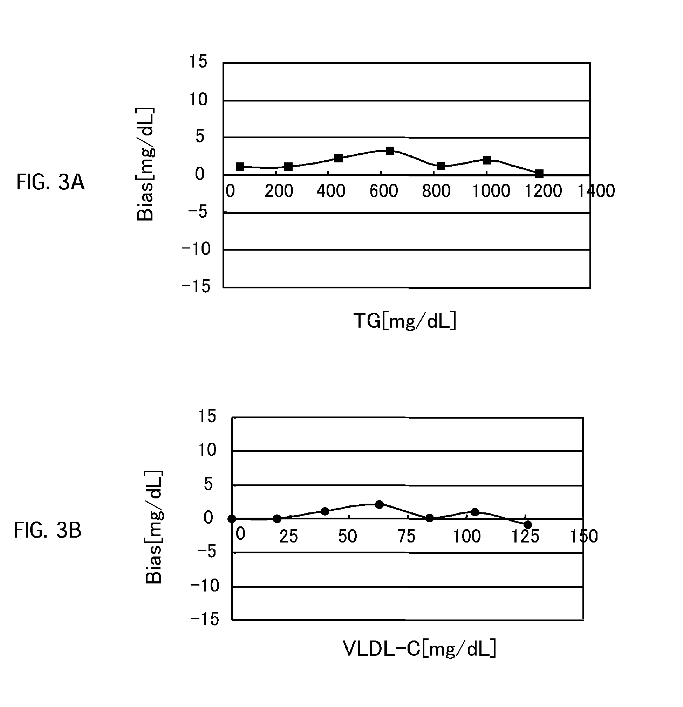

HDL was eliminated by brining HDL into a reaction with enzymes in the same manner as in Reference Example 3 except for changing the concentration of surfactant of the reagent 1-2 to 0.3 wt% (final concentration: 0.053 wt%), and using samples each obtained by adding TG (50 to 1200 mg/dL) or VLDL (0 to 125 mg/dL) to human serum (HDL: 68 mg/dL, LDL: 93 mg/dL) and diluting the resultant with the reagent 1-3 (HDL: 68 mg/dL, TG: 50 to 1200 mg/dL, VDLD: 0 to 125 mg/dL). FIG. 3 shows the results.

FIGS. 3A and 3B are graphs showing one example of results of eliminating HDL in the presence of the surfactants A1 and A2. FIG. 3A shows one example of results of using a sample containing HDL and TG and FIG. 3B shows one example of results of using a sample containing HDL and VLDL. In FIGS. 3A and 3B, the vertical axis indicates the difference from the HDL measured values obtained using a sample free of TG or VLDL and the horizontal axis indicates the concentration of TG or VLDL. As can be seen from FIGS. 3A and 3B, solubilization of TG and VLDL was suppressed adequately when subjecting HDL to an enzyme reaction in the presence of the surfactants A1 and A2.

### (Example 1)

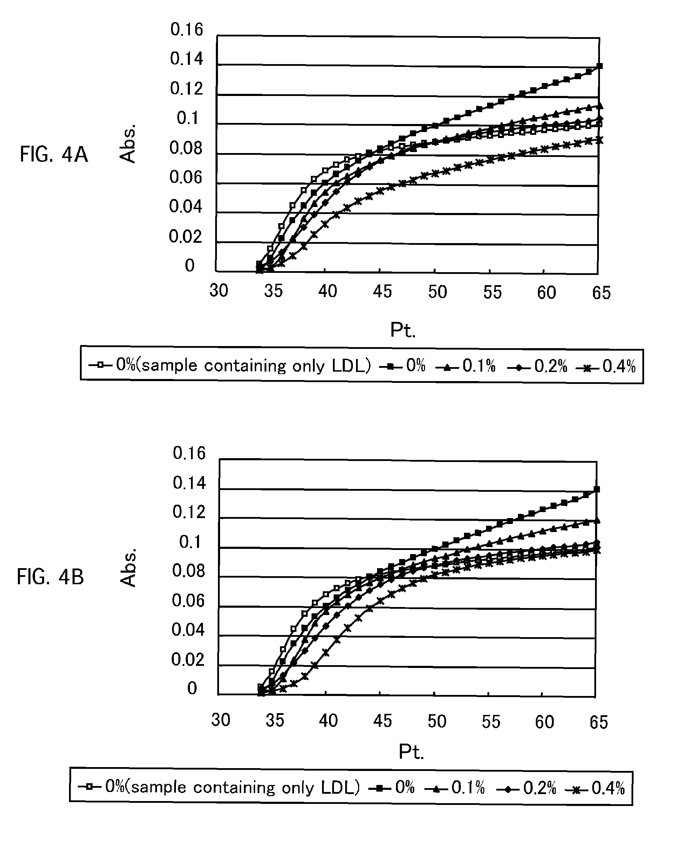

LDL cholesterol was measured using the reagents 1-1 to 1-4 presented in Table 1 above, a surfactant B1 (a polyoxyethylene distyrenated phenyl ether having a HLB of 12.8, trade name: EmulgenA60, manufactured by Kao Corporation, concentration: 8 wt%) and the reagents 2 (three types) containing (any one of) the surfactants presented in Table 5 below. FIGS. 4A and 4B show the results.

LDL cholesterol was measured in the same manner as in Reference Example 1. The concentration of a polyoxyethylene distyrenated phenyl ether having a HLB of 12.8 was 8 wt% (final concentration in the reaction system: 0.71 wt%). As for the surfactants presented in Table 5 below, the concentration was 0 wt%, 0.1 wt%, 0.2 wt% or 0.4 wt%. Two kinds of samples, one only containing LDL (LDL concentration: 50 mg/dL) and the other containing LDL and VLDL (LDL concentration: 52 mg/dL, VLDL: 156 mg/dL), were prepared.

| (Table 5) | | |
|---|---|---|
| Composition | HLB | Trade name |
| polyoxyethylene lauryl ether | 16.9 | Emulgen 123P (manufactured by Kao Corporation) |
| polyoxyethylene myristyl ether | 18.9 | Emulgen 4085 (manufactured by Kao Corporation) |
| polyoxyethylene (35-40) octyl phenyl ether | - | Triton X405 |

FIG. 4A is a graph showing one example of results of using a polyoxyethylene lauryl ether having a HLB of 16.9, and FIG. 4B is a graph showing one example of results of using a polyoxyethylene myristyl ether having a HLB of 18.9. In FIGS. 4A and 4B, the vertical axis indicates absorbance and the horizontal axis indicates measuring time. In FIGS. 4A and 4B, 1 Pt. corresponds to 9 seconds.

As can be seen from FIGS. 4A and 4B, a rise in absorbance with the passage of the measuring time was suppressed with the addition of a polyoxyethylene lauryl ether having a HLB of 16.9 or a polyoxyethylene myristyl ether having a HLB of 18.9. A similar trend was seen when using polyoxyethylene (35-40) octyl phenyl ether (data not shown). Thus, it was shown that the solubilization of VLDL was suppressed and LDL was selectively solubilized with the addition of these surfactants as the surfactant B, thereby allowing the measurement. Therefore, as a result of subjecting LDL to an enzyme reaction in the presence of the surfactants A1, B1, and B2 to solubilize LDL selectively, LDL cholesterol could be solubilized selectively.

As can be seen from FIGS. 4A and 4B, in the case where the concentration of a polyoxyethylene lauryl ether having a HLB of 16.9 or a polyoxyethylene myristyl ether having a HLB of 18.9 was 0.2 wt% (final concentration in the reaction solution: 0.018 wt%), behavior similar to that in the case of the sample free of VLDL (white squares in FIGS. 4A and 4B) was seen. A similar trend was seen in the case of using a polyoxyethylene (35-40) octyl phenyl ether. Thus, it was found that the concentration optimum for these surfactants was 0.2 wt% (final concentration in the reaction solution: 0.018 wt%) under the present conditions.

### (Example 2)

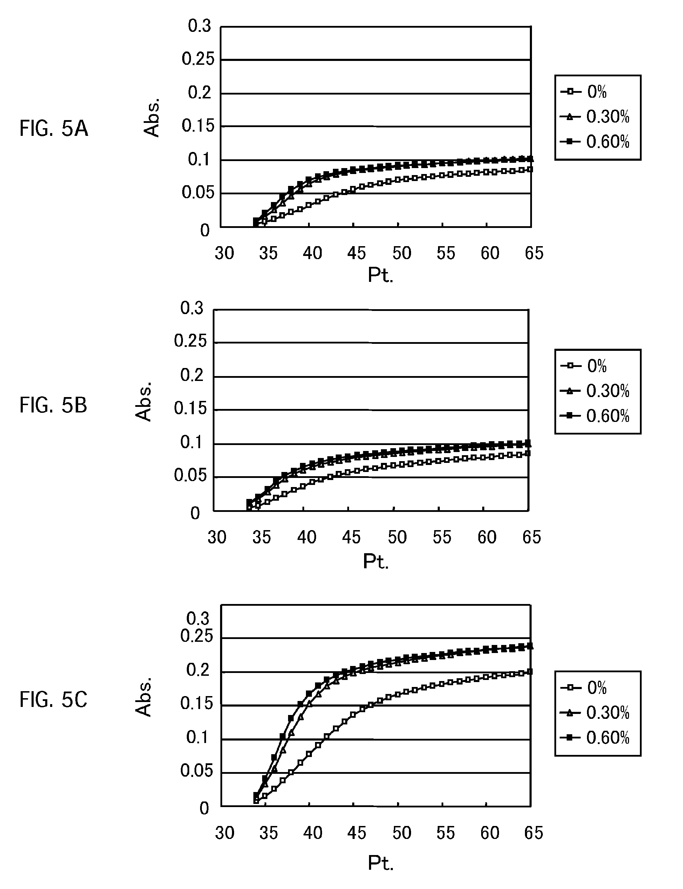

LDL cholesterol was measured in the same manner as in Reference Example 1 using the reagents 1-1 to 1-4 presented in Table 1 above and reagents whose compositions are presented in Table 6 below. FIGS. 5A to 5C show the results. The concentration of the surfactant B3 was 0 %, 0.30 wt% (final concentration in the reaction solution: 0.026 wt%), or 0.60 wt% (final concentration in the reaction solution: 0.053 wt%). A sample having a LDL concentration of 51 mg/dL (VLDL concentration: 0 mg/dL), a sample having a LDL concentration of 136 mg/dL (VLDL concentration: 0 mg/dL), and a sample having a LDL concentration of 49 mg/dL and a VLDL concentration of 156 mg/dL were used.

**(TABLE 6)**

| Composition | Concentration | Final concentration |
|---|---|---|
| surfactant B1 *¹ | 8 wt% | 0.71 wt% |
| surfactant B2 *² | 0.2 wt% | 0.02 wt% |
| surfactant B3 *³ | 0 to 0.6 wt% | 0 to 0.05 wt% |

| | | |
|---|---|---|
| *¹ polyoxyethylene distyrenated phenyl ether (trade name: Emulgen A60, manufactured by Kao Corporation, HLB:12.8) *² polyoxyethylene myristyl ether (trade name: Emulgen 4085, manufactured by Kao Corporation, HLB:18.9) *³ polyoxyethylene lauryl ether sodium sulfate (trade name: Emal 20C, manufactured by Kao Corporation) or lauryl benzene sodium sulfate (trade name: lauryl benzene sodium sulfonate) | | |

FIG. 5A is a graph showing one example of results of using the sample having a LDL concentration of 136 mg/dL, FIG. 5B is a graph showing one example of results of using the sample having a LDL concentration of 51 mg/dL, and FIG. 5C is a graph showing one example of results of using the sample having a LDL concentration of 49 mg/dL and a VLDL concentration of 156 mg/dL. In FIGS. 5A to 5C, the vertical axis indicates absorbance, the horizontal axis indicates measuring time, and 1 Pt. corresponds to 9 seconds.

As can be seen from FIGS. 5A to 5C, it was shown that the solubilization of LDL could be accelerated with the addition of polyoxyethylene lauryl ether sodium sulfate or lauryl benzene sodium sulfate. Therefore, it was shown that LDL cholesterol could be measured selectively and effectively by carrying out the enzyme reaction in the presence of the surfactants A1, B1, B2 and B3.

### (Example 3)

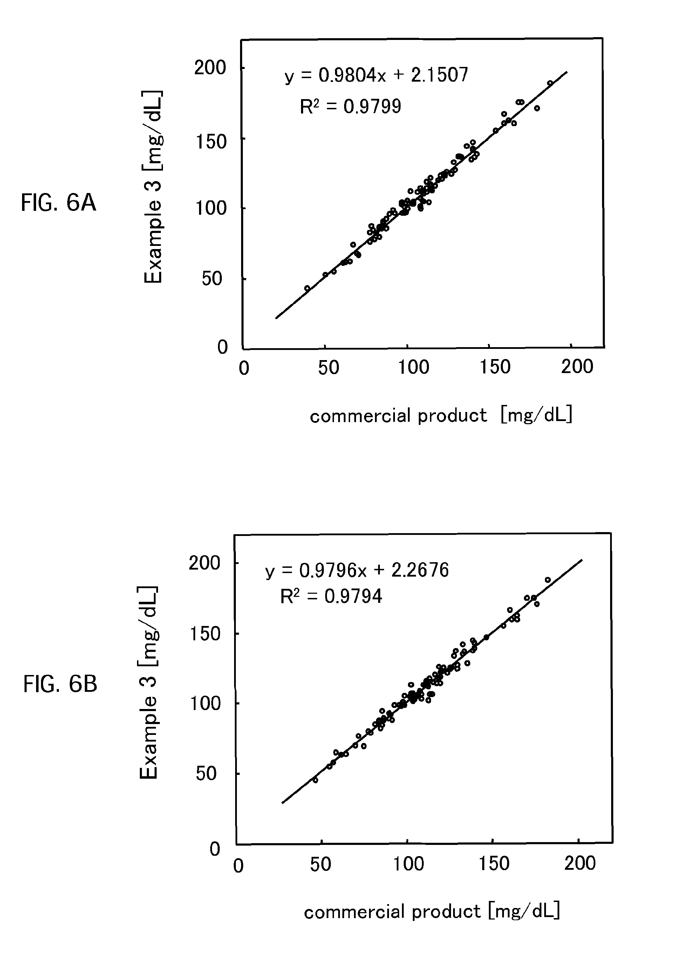

LDL cholesterol was measured in the same manner as in Reference Example 1 except for using reagents 1-1 to 1-4 and 2 presented in Table 7 below and a routine test specimen as a sample, and obtained measurement values were compared with those obtained by using commercially available LDL cholesterol measuring reagents. FIGS. 6A and 6B show the results. As the commercially available measuring reagents, Determiner LDL (trade name, manufactured by Kyowa Medex Co., Ltd.) and CholetestLDL (trade name, manufactured by Sekisui Medical Co., Ltd.) were used. The routine test specimen refers to an actual sample routinely handled in test centers and hospital examination rooms.

| (Table 7) | Composition | Concentration | Final concentration |
|---|---|---|---|
| Reagent 1-1 | CHDH | 7.5 U/mL | 4.0 U/mL |
| | Diaphorase | 7.5 U/mL | 4.0 U/mL |
| | BES (pH 7.0) | 300 mM | 159 mM |
| Reagent 1-2 | surfactant A1 *¹ | 1.1 wt% | 0.19 wt% |
| | surfactant A2 *² | 0.3 wt% | 0.053 wt% |
| | CE | 3 U/mL | 0.53 U/mL |
| | BES (pH 7.0) | 800 mM | 141 mM |
| Reagent 1-3 | Tris-HCl (pH 9.0) | 400 mM | 44.7 mM |
| Reagent 1-4 | phosphate buffer (pH 6.0) | 20 mM | 1.76 mM |
| | MTT | 2mM | 0.18 mM |
| | NAD | 10 mM | 0.88 mM |
| Reagent 2 | surfactant B1 *³ | 8% | 0.71 % |
| | surfactant B2 *⁴ | 0.2% | 0.02% |
| | surfactant B3 *⁵ | 0.6% | 0.053 % |

| | | | |
|---|---|---|---|
| *¹ polyoxyalkylene polycyclic phenyl ether: Newcol 2608 F (trade name: manufactured by Nippon Nyukazai Co., Ltd., HLB 12.5, the product includes oxyethylene and oxypropylene as oxyalkylene) *² polyoxyethylene distyrenated phenyl ether: Emulgen A500 (trade name: manufactured by Kao Corporation, HLB 18.0) *³ polyoxyethylene distyrenated phenyl ether: Emulgen A-60 (trade name: manufactured by Kao Corporation, HLB 12.5) *⁴ polyoxyethylene myristyl ether: Emulgen 4085 (trade name: manufactured by Kao Corporation, HLB 18.9) *⁵ alkyl benzene sodium sulfonate: lauryl benzene sodium sulfonate (manufactured by Nacalai Tesque Inc.) | | | |

FIG. 6A is a graph showing one example of correlation with Determiner LDL (trade name, manufactured by Kyowa Medex Co., Ltd.), and FIG. 6B is a graph showing one example of correlation with Choletest LDL (trade name, manufactured by Sekisui Medical Co., Ltd.). In FIGS. 6A and 6B, the vertical axis indicates measurement values of LDL cholesterol in Example 3 and the horizontal axis indicates measurement values of LDL cholesterol obtained by using the commercially available cholesterol measuring reagents.

As can be seen from FIGS. 6A and 6B, the measurement values of LDL cholesterol obtained by using the reagents of Example 3 and those obtained by using the commercially available cholesterol measuring reagents had favorable correlation, where the correlation function with the measurement values obtained by using Determiner LDL was 0.9799, and the correlation function with the measurement values obtained by using Choletest LDL was 0.9794. Therefore, the results showed that LDL cholesterol can be measured by the LDL cholesterol measurement method of the present invention with the same precision as that of the commercially available LDL measuring reagents.

### Industrial Applicability

The sample analysis method of the present invention is useful in a variety of fields such as medical and clinical testing fields, for example.

## Claims

1. A method for measuring cholesterol in low-density lipoproteins in a sample, the method comprising a step (I) of subjecting the cholesterol in low-density lipoproteins to an enzyme reaction in the presence of surfactants A1, B1, and B2 in a reaction solution containing the sample treated to eliminate high-density lipoproteins,
wherein the surfactant A1 is polyoxyalkylene polycyclic phenyl ether having HLB of 12.5 or less,
the surfactant B1 is at least one selected from the group consisting of polyoxyethylene polycyclic phenyl ether having HLB of 12.6 or more, polyoxyethylene distyrenated phenyl ether having HLB of 12.7 or more and 14.5 or less, and polyoxyethylene styrenated phenyl ether having HLB of 12.0 or more and 14.5 or less, and
the surfactant B2 is at least one selected from the group consisting of polyoxyethylene lauryl ether having HLB of 16.9 or more, polyoxyethylene myristyl ether, and polyoxyethylene (35-40) octyl phenyl ether.

2. The measurement method according to claim 1, wherein the elimination of the high-density lipoproteins includes mixing the surfactant A1 and a surfactant A2 with the sample to subject cholesterol in the high-density lipoproteins in the sample to an enzyme reaction, and
the surfactant A2 is at least one selected from the group consisting of polyoxyethylene distyrenated phenyl ether having HLB of 18.0 or more, polyoxyethylene lauryl ether having HLB of 16.9 or more, and polyoxyethylene myristyl ether.

3. The measurement method according to claim 1 or 2, wherein the step (I) includes adding a surfactant B3 to the reaction solution containing the sample treated to eliminate the high-density lipoproteins to subject the cholesterol in the low-density lipoproteins to the enzyme reaction in the presence of the surfactants A1, B1, B2 and B3 in the reaction solution, and
the surfactant B3 is at least one of polyoxyethylene alkyl ether sulfate and alkyl benzene sulfonate.

4. The measurement method according to any one of claims 1 to 3, wherein the polyoxyalkylene polycyclic phenyl ether is polyoxyethylene polyoxypropylene polycyclic phenyl ether.

5. The measurement method according to any one of claims 1 to 5, wherein the enzyme reaction in the step (I) includes subjecting the low-density lipoproteins in the sample to an enzyme reaction with cholesterol dehydrogenase and cholesterol esterase.

6. A kit for measuring cholesterol in low-density lipoproteins, the kit comprising:
a surfactant A1, the surfactant A1 being polyoxyalkylene polycyclic phenyl ether having HLB of 12.5 or less;
a surfactant B1, the surfactant B1 being at least one selected from the group consisting of polyoxyethylene polycyclic phenyl ether having HLB of 12.6 or more, polyoxyethylene distyrenated phenyl ether having HLB of 12.7 or more and 14.5 or less, and polyoxyethylene styrenated phenyl ether having HLB of 12.0 or more and 14.5 or less; and
a surfactant B2, the surfactant B2 being at least one selected from the group consisting of polyoxyethylene lauryl ether having HLB of 16.9 or more, polyoxyethylene myristyl ether, and polyoxyethylene (35-40) octyl phenyl ether.

7. The kit according to claim 6, further comprising a surfactant A2, the surfactant A2 being at least one selected from the group consisting of polyoxyethylene distyrenated phenyl ether having HLB of 18.0 or more and polyoxyethylene myristyl ether.

8. The kit according to claim 6 or 7, further comprising a surfactant B3, the surfactant B3 being at least one of polyoxyethylene alkyl ether sulfate and alkyl benzene sulfonate.

9. The kit according to any one of claims 6 to 8, further comprising a cholesterol measuring reagent.

10. The kit according to claim 9, wherein the cholesterol measuring reagent includes cholesterol dehydrogenase and cholesterol esterase.

11. The kit according to claim 10, wherein the kit includes a first reagent containing the cholesterol dehydrogenase, a second reagent containing the cholesterol esterase, the surfactants A1 and A2, and a third reagent containing the surfactant B1.
